# EUROPEAN PATENT APPLICATION

(11) **EP 4 014 830 A1**
(43) Date of publication of application: **22.06.2022**
(21) Application number: 20215590.9
(22) Date of filing: 18.12.2020
(51) Int. Cl.: A61B 1/00

(54) **AN ENDOSCOPE AND METHODS OF MANUFACTURE**

(71) Applicant: Ambu A/S, 2750 Ballerup (DK)
(72) Inventor: MATTHISON-HANSEN, Kaspar Mat, 3000 Helsingør (DK)
(74) Representative: AWA Denmark A/S

(57) **Abstract**

An endoscope comprising:
an elongate insertion tube; and
a working channel extending through the insertion tube to allow for insertion of a retractable instrument into a body and/or for suction of fluids from the body through the working channel;
wherein the working channel comprises an at least partly extruded or at least partly coextruded working channel tube, the working channel tube comprising an extruded inner layer of an inner layer material and an extruded outer layer of an outer layer material.

## Description

### Technical field

The present disclosure relates to endoscopes and working channel tubes for endoscopes as well as methods of manufacture thereof.

### Background

Endoscopes are well known for visually inspecting places that are difficult to access, such as human body cavities. Typically, the endoscope comprises an elongated insertion tube with an operating handle at the proximal end, as seen from the operator, and visual inspection means, such as a built-in camera, at the distal end of the elongated insertion tube. This definition of the terms distal and proximal, i.e. proximal being the end closest to the operator and distal being the end remote from the operator, as used herein for endoscopes in general, is adhered to in the present specification.

As the name indicates, endoscopes are used for seeing inside things, such as lungs or other human body cavities of a patient. Endoscopes are therefore typically equipped with a light source and a vision receptor including a vision sensor, such as a camera or an image sensor. Instead of using cameras, endoscopes may also be fiber-optic, in which case the optical fibers run along the inside of the elongated insertion tube to the tip part assembly. Provided that enough light is present, it is possible for the operator to see where the endoscope is steered and to set the target of interest once the tip has been advanced thereto.

Electrical wiring for the camera and other electronics typically runs along the inside of the elongated insertion tube from the handle to the tip part assembly.

For some endoscope applications, a working channel of an endoscope runs along the inside of the insertion tube from the handle to the tip part assembly, e.g. allowing liquid to be removed from the body cavity or allowing for insertion of surgical instruments or the like, into the body cavity. A tip of an elongated tool or instrument of small cross-sectional extent can be inserted into a port positioned e.g. at or in the endoscope handle and further pushed through the working channel to exit at an exit port e.g. provided at a tip of the endoscope. Having exited the exit port, the tip of the tool or instrument performs a function, such as retrieval of a tissue sample, inside a body cavity of a patient. During such an operation, parts of the tool or instrument including its tip will contact an inner surface of the working channel during movement in the working channel. Especially when the tip of the tool or instrument is inserted into the body cavity, a high dynamic and/or static friction between the tool or instrument and an inner surface of the working channel or changes in friction along a length of the working channel may influence precision of the operation of the tool or instrument and may even lead to damage of the body.

In cases where the working channel is used for suction, the working channel can generally be denoted, also or alternatively, "a suction channel". The suction channel may be connected to a suction connector, typically positioned at a handle at the proximal end of the insertion tube. Such a working channel may comprise or consist of a working channel tube which in case of suction applications can be denoted a "suction tube".

Some working channel tubes consist of two or more tube portions assembled or joined by a sleeve or collar.

To be able to maneuver the endoscope inside the body cavity, the distal end of the endoscope may comprise a bending section with increased flexibility, e.g. an articulated tip part assembly allowing the operator to bend this section. Typically, this is done by tensioning or slacking steering wires also running along the inside of the elongated insertion tube from the articulated tip part assembly to a control mechanism of the handle.

Additives in the material forming the working channel tube may reduce friction in working channels for endoscopes; however, such known solutions may only to some extent reduce the friction as desired.

WO2016/188543A1 discloses an endoscope comprising a working channel extending inside an insertion tube all the way from a distal tip part to an operating handle. The working channel comprises several tubes of different flexibility and connected by a connecting tube member. The tubes can be made of different polyurethanes, such as Pellethane^{®} in different variants.

On this background, it may be seen as an object of the present disclosure to provide endoscopes comprising an alternative or improved working channel tube. Such an endoscope and/or working channel tube may mitigate one or more drawbacks, one or more of which may be realized by a person skilled in the art in view of the background described above.

The term "endoscope" may be understood herein as a device suitable for examination of natural and/or artificial body openings, e.g. for exploration of a lung cavity. Additionally, or alternatively, the term "endoscope" may be defined as a medical device.

In this disclosure, as is common to medical devices such as endoscopes, a proximal-distal direction may generally be defined as an axis or direction extending along an insertion tube of the endoscope, adhering to the definition of the terms distal and proximal, i.e. proximal being the end closest to the operator and distal being the end remote from the operator. The proximal-distal direction need not necessarily be straight; for example, if the insertion tube is bent, the proximal-distal direction may follow a curvature of the insertion tube. The proximal-distal direction may for instance extend along a center line of the insertion tube. A distal end of a tip part assembly of an endoscope may form a distal end of the endoscope.

### Summary

A first aspect of this disclosure relates to an endoscope comprising:
an elongate insertion tube; and
a working channel extending through the insertion tube to allow for insertion of a retractable instrument into a body and/or for suction of fluids from the body through the working channel;
wherein the working channel comprises an at least partly extruded or at least partly coextruded working channel tube, the working channel tube comprising an extruded inner layer of an inner layer material and an extruded outer layer of an outer layer material.

Potential advantages of the endoscopes of the present disclosure, especially the working channel tubes thereof, include reduction of friction towards tools or instruments passing through the working channel and/or reduction of a tendency of the working channel or working channel tube to kink during operation.

Another advantage may involve that the outer layer of the working channel tube stabilizes the working channel tube and/or reduces kink, while the inner layer potentially provides low friction on an interior surface thereof.

With the working channel tubes of the endoscopes of the present disclosure, joints and/or assembly of tube parts may be avoidable, while potentially providing or maintaining advantageous properties of the working channel tube.

The endoscopes of the present disclosure, especially the working channel tubes thereof, may be made to be more environmentally friendly than prior art endoscopes and/or may be manufactured more readily and more environmentally friendly, may comprise fewer parts and/or may be manufactured with lower costs than prior art endoscopes, potentially in a simpler process and/or with lower material costs and/or with reduced use of materials.

Other advantages may involve the provision of improved electrical insulation of the working channel tube from the outside, thus mitigating the risk of an insulation breakdown and a resulting excessive leakage current and/or provision of liquid-tightness to mitigate liquid ingress into parts of the endoscope inserted into a body and, especially, into any electrical or optical components of the endoscope.

Other advantages may involve the provision of endoscopes assembled from fewer parts, which may allow simpler manufacture. Avoidance of joints or assemblies of working channel tubes may contribute to this advantage. Other advantages may involve the provision of a smaller diameter or cross-sectional extent of parts of the endoscope.

Another advantage may involve that material stickiness of the inner layer and/or outer layer of the working channel tube is reduced.

Either of the inner layer and the outer layer may be an extruded monolayer or may be an inner and outer, respectively, sub-layer of a coextrusion. A coextrusion is to be understood herein as several layers coextruded together or with each other. A monolayer is to be understood herein as a single-layer extruded layer, i.e. not a coextrusion. The term "an extruded layer" as used herein is to be understood as including both a monolayer and a (sub-)layer of a coextrusion and may comprise only one single layer or one single sub-layer, respectively.

The inner layer material may provide the inner layer with an inner low-friction surface, and/or the inner layer material may be a low-friction material. The outer layer may be a support layer, potentially for providing structural integrity to the working channel tube.

The inner layer material and/or the outer layer material be a thermoplastic material, such as a thermoplastic polymer or a thermoplastic polymer material.

A coil may be positioned between two layers and may be embedded, such as positioned in a track, in a surface one or both layers and may potentially not extend through a total thickness of one or both of these two layers. These two layers may comprise or consist of one and the same material, including the materials disclosed above.

In some embodiments, the outer layer material is a thermoplastic polyurethane having a Shore A hardness according to ASTM D2240-15 within a range of 60 to 100. The outer layer material can be of or can comprise other materials, such as other thermoplastic polymers.

In some embodiments, the inner material is a high-density polyethylene. The inner layer material can be of or can comprise other materials, such as other thermoplastic polymers.

A ratio of a wall thickness of the outer layer in relation to a wall thickness of the inner layer can be at least 1.5, 2, 2.5, 3, 3.5, 4, 5, 6, 7, 8, 9, 10, 11, 12, 13, 14, 15, 16, 17, 18, 19, or 20.

The inner layer material may provide the inner layer with an inner low-friction surface, and/or the inner layer material may be a low-friction material. The outer layer may be a support layer, potentially for providing structural integrity to the working channel tube.

In some embodiments, the inner layer material is selected to provide a low surface friction of an inner surface of the inner layer towards the retractable instrument, and/or the outer layer material is a support layer for prevention of kinking of the working channel tube.

In some embodiments, the outer layer material is a thermoplastic polyurethane.

The polyurethane (PU) of or the outer layer material may be a coextrudable and/or thermoplastic PU elastomer, may have a hardness according to any one of the hardness values as disclosed herein for the outer layer and/or may have a specific gravity according to ASTM D 792 of 1.1 to 1.2, a Tensile modulus according to ASTM D 412 of 6 to 7 MPa (50 % elongation) and/or of 9 to 11 MPa (100 % elongation) and/or of 18 to 20 MPa (300 % elongation), an ultimate tensile strength according to ASTM D412 of 35 to 45 MPa, an ultimate elongation according to ASTM D412 of 500 to 600 %, an elongation set after break according to ASTM D412 of 50 to 70 %, a tear strength (Die C) according to ASTM D624 of 90 to 100 MPa, compression set according to ASTM D 395 (Method B) of 20 to 30 % (25°C) and/or 35 to 45 % (70°C), A Taber abrasion resistance according to ASTM D 1044 (1000 g, 1000 cycles, H-22 wheel (coarser)) of 40 to 70 mg, a flexural modulus according to ASTM D 790 of 65 to 75 MPa, a vicat softening temperature according to ASTM D 1525 of 85 to 95°C, a coefficient of linear thermal expansion according to ASTM D 696 of 150 to 170 10⁻⁶ mm/mm/°C, a glass transition temperature according to DSC of -30 to -36°C, and/or a melt index according to ASTM D 1238 (224°C, 1.2 kg load) of 30 to 35 g/10 m. Experiments have shown that such polyurethanes are especially suitable for embodiments where the outer layer is a monolayer.

Other suitable PU types may be aliphatic polyether-based thermoplastic polyurethanes and/or can have a hardness according to any one of the hardness values as disclosed herein for the outer layer and/or can have a specific gravity according to ASTM D792 of 1.06 to 1,10 g/cc and/or a linear mold shrinkage according to ASTM D955 of 0.0050 to 0.0012 cm/cm and/or a hardness shore A of 85 to 89 and/or tensile strength, ultimate according to ASTM D412 of 50 to 55 MPa and/or an elongation at break according to ASTM D412 of 380 to 400 % and/or a 100% modulus according to ASTM D412 of 6.8 to 7 MPa (at strain 100 %) and/or a 200% modulus according to ASTM D412 of 12 to 14 MPa (at strain 200 %) and/or a 300% modulus according to ASTM D412 of 28 to 31 MPa (at strain 300 %) and/or a flexural modulus according to ASTM D790 of 0.02 to 0.05 MPa. Experiments have shown that the latter polyurethanes are especially be suitable in embodiments where the outer layer contacts a contact layer of similar material, the contact layer being a layer of a coextrusion that also comprises the inner layer, and wherein a coil is potentially embedded between the outer layer and the contact layer, see also elsewhere herein. Such polyurethanes may further comprise barium sulphate, such as 10 to 50, 15 to 25, or 35 to 45 wt% barium sulphate, the remaining parts of the material potentially being substantially of any one of the compositions as described in the present paragraph and/or in the previous paragraph. A sub-layer or an outer sub-layer of a coextrusion comprising the inner layer may additionally, or alternatively, be of a material as described in this paragraph.

The outer layer may consist of, potentially except for unavoidable impurities and/or additives, or may mainly comprise polyurethane or a polyurethane, such as comprise at least 50, 50, 70, 80, 90, 95, 98, 99, or 100 w% PU.

In some embodiments, the outer layer material has a Shore A hardness according to ASTM D2240-15 within a range of 60 to 100.

In this disclosure, Shore A and Shore D hardnesses are determined according to ASTM D2240-15 "Standard Test Method for Rubber Property - Durometer Hardness" as published in January 2016. The Shore hardness may generally be determined from the material of the layer in question before or after extrusion of the material to form a layer of a working channel tube, i.e. when the material is in a solid form, whether this form is crystallized or amorphous. Before extrusion of the layer, the Shore hardness of the layer material, i.e. the layer raw material, such as polymer pellets, that is to be extruded to form the extruded layer can be measured. After extrusion, the layer material of an extruded layer may be collected from the working channel tube before determination of the Shore hardness of the layer material. A person skilled in the art will be able to devise methods for the provision of a suitable test specimen from layer raw material and layers of working channel tubes of embodiments of the endoscopes of the present disclosure for determination of the Shore A hardness. For example, according to ASTM D2240, a test specimen may be composed of plied pieces to obtain the necessary thickness of the test specimen, such as to produce a sandwich structure. Such pieces to be plied can be cut from the working channel tube. Similarly, the inner and outer layers may be delaminated from each other just by pulling them apart or, if necessary, by cutting the layers from each other. If relevant, an amount of a layer material for or of a working channel tube can be assembled, extruded, molded, stacked, and/or otherwise treated to form a specimen in a shape and size for suitable determination of the Shore hardness. Another option is to suitably determine Shore hardness of only one or only some of the extruded layers of the tube wall as well as of the entire tube wall and, then, determine the Shore hardness of one or more remaining layers based on these determinations. The values of Shore hardnesses as disclosed herein may be converted by means of suitable calculations to other of the Shore scales, so that a value or range of Shore A hardness may, for example, be converted to the Shore D scale. Another alternative is to determine Young's modulus E of the material in question and calculate the relevant Shore hardness from that value. A person skilled in the art will be able to devise how to make such calculations.

The Shore A hardness of the outer layer or of the outer layer material may be 85 to 97, 85 to 95, 86 to 94, 87 to 93, 88 to 92, or 89 to 91, such as 90. Additionally, or alternatively, the Shore A hardness of the outer layer or of the outer layer material may be above 65, 70, 75, 80, 85, 90, or 95 and/or may be below 95, 90, 85, 70, or 65. Additionally, or alternatively, the Shore A hardness of the outer layer or of the outer layer material may be 80 to 100, 85 to 100, 85 to 95, or 90 to 95.

The Shore D hardness of the inner layer or of the inner layer material may be 40 to 80, 45 to 75, 50 to 70, 55 to 70, or 60 to 65, such as 63.

In an embodiment, the outer layer material is a thermoplastic polyurethane having a Shore A hardness according to ASTM D2240-15 within a range of 85 to 100.

In an embodiment, the inner layer material is a high-density polyethylene.

A high-density polyethylene or HDPE or PEHD may be defined as a polyethylene having a density of 930 to 970 kg/m³.

The HDPE of the inner layer material and/or the inner layer material of the working channel tube of the endoscopes of this disclosure may have a density of at least 930, 935, 940, 950, 955, 956, 957, or 958 kg/m³ and/or equal to or less than 970, 965, 962, 961, 960, 959, or 958 kg/m³. Any ranges combining these numbers may be envisaged. The density values in this context may be according to ISO 1183.

The Shore D hardness of the HDPE of the inner layer or of the inner layer material may be 40 to 80, 45 to 75, 50 to 70, 55 to 70, or 60 to 65, such as 63.

The HDPE of the inner layer material and/or the inner layer material of the working channel tube may have a melt index of 0.2 to 0.4 g/10 min according to ISO 1133, a flexural modulus of 1300 to 1500 MPa according to ISO 178, a tensile modulus of 1200 to 1400 according to ISO 527-2, a tensile strain at yield (50 mm/min) of 6 to 10 % according to ISO 527-2, and/or a tensile strength at yield (50 mm/min) of 27 to 32 MPa according to ISO 527-2. The HDPE of the inner layer material and/or the inner layer material of the working channel tube may be a coextrudable and/or bimodal HDPE and/or may have a hardness as indicated herein for the inner layer.

The inner layer may consist of, potentially except for unavoidable impurities and/or additives, or may mainly comprise polyethylene or a polyethylene or HDPE or a HDPE, such as comprise at least 50, 50, 70, 80, 90, 95, 98, 99, or 100 w% PE.

Generally, each of the inner and outer layers may comprise one or more additives, such as barium sulphate (BaSO₄), which may be particularly relevant for the outer layer. Additives like BaSO₄ may reduce stickiness of the working channel tube, which may be an advantage when threading the working channel tube into the insertion tube during the manufacturing process of the endoscopes of the present disclosure. When adding barium sulphate, the tube material may no longer be radio opaque when watched on X-ray.

The inner and outer layers as well as the working channel tube may, generally, have substantially unchanged associated wall thicknesses along an entire length of the working channel tube.

Endoscopes with working channels come in many different sizes depending on the intended use thereof, and the thickness of the outer layer may vary with or be set according to the outer diameter of the working channel tube. Generally, in the endoscopes according to the present disclosure, the thickness of the inner layer in different sizes and variations may be substantially the same independently of the outer diameter of the working channel and the thickness of the outer layer.

Generally, a wall thickness of the inner layer may be within a range of 0.01 to 0.1, 0.02 to 0.08, 0.03 to 0.07, or 0.04 to 0.06 mm, such as 0.05 mm.

Generally, a wall thickness of the outer layer may be within a range of 0.1 to 0.5 mm.

In an exemplary working channel with an inner diameter of 2.2 mm and an outer diameter of 2.7 mm, the outer layer is 0.2 mm. In another exemplary working channel tube with an inner diameter of 2.8 mm and an outer diameter of 3.3 mm, the outer layer is also 0.2 mm. In another exemplary working channel with an inner diameter of 4.3 mm and an outer diameter of 5.2 mm, the inner layer has a thickness of 0.05 mm and the outer layer has a thickness of 0.4 mm. In case the outer layer is part of a coextrusion including two or more sub-layers, a total wall thickness of the coextrusion, i.e. including all sub-layers, may be as disclosed herein for the wall thickness of the outer layer itself. If there are a total of two sub-layers of the coextrusion comprising the outer layer, the outer layer may have a larger thickness than an inner sub-layer of the coextrusion, such as 1.5, 2, 2.5, 3, 3.5, 4, 4.5, or 5 times the wall thickness of the inner sub-layer.

Generally, any one or more of the dimensions or ranges of dimensions as mentioned herein may be combined.

Generally, when coextruded, polyethylene, polyurethane and/or other layer materials may not bond or may not bond so well to each other so that delamination of the inner and outer layers may occur during use, depending on the PE and PU used. However, experiments have shown that mechanical friction between two layers of the working channel tubes of the endoscopes of the present disclosure can be made to sufficiently keep the two layers in place during use of the endoscope. For example to ensure that movement of a tool through the working channel tube is, nonetheless, not hindered if the layers should delaminate from each other, at a proximal end of the endoscope, e.g. adjacent a working channel opening or entry port at an operating handle, a proximal portion of the working channel tube may be everted and positioned in an assembly part accommodating the everted tube portion. This entry port to the working channel may include a connector or pipe connection or branch piece connected to the working channel tube.

Alternatively, the polyethylene, polyurethane, and other layer materials may bond so well to each other that delamination of the inner and outer layers can be expected to not occur during normal use, which may especially be achieved by the inclusion of one or more layers and/or tie layers between the inner and outer layers as disclosed herein. In the case of extrusion coating, an extrusion coating primer can be applied to the surface on which the coating is applied. In these and other cases, the working channel tube may proximally be secured at and/or to the operating handle and/or the working channel port by means of gluing and/or application of a glue or adhesive between the two parts to secure them to each other. Distally, the working channel tube may be attached, e.g. to a tip part assembly of the endoscope and/or a housing thereof, also by means of gluing and/or application of a glue or adhesive between the two parts to secure them to each other.

Generally, the working channel tubes of the endoscopes of the present disclosure may consist only of extruded and/or coextruded layers and/or may consist of two, three, four, or more extruded or coextruded layers, potentially except for a coil, such as a helical coil, or a spring which may prevent kink of the working channel tube. Such a coil or spring may be provided or embedded, such as in one or more potentially cut tracks, in one layer of the tube or in two adjacent layers of the tube, and/or may be provided between two layers of the tube. The working channel tube may not be secured to other parts of the endoscope in any other positions thereof, which may provide improved flexibility of the working channel tube during use of the endoscope. The glue or adhesive may especially be in contact with the outer layer of the working channel tube. Experiments have shown that due to the improved strength of the gluing connection, gluing of the outer layer including PU is preferred over gluing of the inner layer including PE. Therefore, it may be the outer layer of the working channel tube that is glued or adhered to secure the working channel tube.

Generally, the working channel tubes of the endoscopes of the present disclosure may not include any coextrusion tie layers between the inner and outer layers. The working channel tube may include one, two, or more coextrusion tie layers between the inner and outer layers. A wall thickness of the tie layer or tie layers, or a total thickness of the tie layers in case more than one tie layer is provided, may be small, such as less than half, 0.4, 0.3, 0.2, or 0.1 a thickness of the inner layer. A suitable material for the coextrusion tie layer or coextrusion tie layers may be a maleic anhydride grafted linear low-density polyethylene and/or may have a melt index according to ISO 1133/ASTM D1238 of 2 to 2.5 g/10 min and/or a melting point according to ISO 11357-3 of 110 to 130°C and/or a Vicat softening temperature (10N; on compression molded samples) according to ISO 306/ASTM D1525 of 80 to 90° and/or a density according to ISO 1183/ASTM D1505 of 0.90 to 0.92 g/m³.

Generally, the working channel tubes of the endoscopes of the present disclosure may not comprise any non-extruded or non-coextruded layers, such as coatings and/or surface treatments.

Generally, the working channel tubes of the present disclosure may be flexible. Suitable flexibility may be achieved by providing the layers of the working channel tube as disclosed herein.

Generally, in endoscopes of the present disclosure, the working channel tube may be the only working channel tube of the endoscope.

Generally, in the endoscopes of the present disclosure, each of the inner and outer layers may extend along a substantially entire length of the working channel tube.

Generally, the working channel and/or working channel tube may have a length or an entire length of at least 500, 600, 700, 800, 900, 1000, 1100, 1200, 1300, 1300, 1400, 1500, 1600, 1700, 1800, 1900, or 2000 mm. The working channel and/or working channel tube may extend from an entry port of the endoscope in or at or on an operating handle of the endoscope to the working channel at a distal end of the endoscope and/or of a tip part assembly of the endoscope and/or to a tip part assembly of the endoscope to an operating handle of the endoscope. The working channel and/or working channel tube may extend through an entire length of the insertion tube and/or through a bending section of the endoscope.

In some ebmodiments, no further working channel tubes are included in the endoscope besides the working channel tube.

In an embodiment, the working channel includes only one single working channel tube, which working channel tube is the working channel tube.

Generally, in the endoscopes of the present disclosure, the working channel and/or working channel tube may be substantially tubular and/or have a circumferentially extending, potentially substantially cylindrical or circular cylindrical, outer wall enclosing an inner working channel spacing.

Generally, in the endoscopes of the present disclosure, an inner diameter of the working channel tube may be above 1, 2, 3, 4, 5, 6, or 7 mm and/or below 10, 8, 7, 6, or 5 mm, such as 1 to 10 mm, 2 to 8 mm, 3 to 8 mm, or 4 to 8 mm.

Generally, in the endoscopes of the present disclosure, the working channel tube may have an inner diameter of 0.5 to 5 mm, 2 to 4 mm, or 2.5 to 3.5 mm.

Generally, in the endoscopes of the present disclosure, a wall thickness of a circumferential wall of the working channel tube may be 0.1 to 0.5, 0.2 to 0.4, or 0.2 to 0.3 mm.

In an embodiment, the working channel tube consists of, in sequence, the inner layer, a tie layer, a contact layer, and the outer layer as well as a coil located between the contact layer and the outer layer, potentially as described elsewhere herein. The coil may extend into one of or into both of the contact layer and the outer layer, potentially in a track or tracks in an outer surface of the associated layer. In such embodiments, the inner layer and tie layer can have an accumulated wall thickness of 0.0635 mm, an inner of the two sub-layers facing and in contact with the tie layer can have a wall thickness of 0.0635 mm, and a total wall thickness of the outer layer can be 0.425 mm. The coil can be a stainless steel 304V spring having a wire thickness or diameter of 0.152 mm and a WPI (Wraps Per Inch) of 60. The inner layer is a HDPE with the characteristics as described above, the tie layer can be of a material with the characteristics described above for a suitable tie layer material, and the contact layer and the outer layer can be of the PU indicated to be suitable for this structure of the outer layer. An inner diameter of the working channel tube can be 4.3 mm, and an outer diameter thereof can be 5.15 mm. A wall thickness of the tie layer can be small, such as less than 0.5, 0.4, 0.3, 0.2, or 0.1 a thickness of the inner layer.

Generally, in the endoscopes of the present disclosure, the working channel may comprise or consist of a distal portion, which may potentially be provided in one piece with a housing of the tip part assembly, and which may only extend from a distal end of the working channel and/or a tip part assembly to a proximal end of the tip part assembly, and a proximal portion interconnected to and/or abutting the distal portion, wherein the proximal portion may be the working channel tube. This distal portion may have substantially the same internal diameter as the working channel tube. These distal and proximal portions may be interconnected by means of e.g. an adhesive.

Generally, in the endoscopes of the present disclosure, the working channel and/or working channel tube can have a longitudinally extending center line, which may extent in the proximal-distal direction, potentially from a proximal end to a distal end of the working channel and/or the working channel tube and/or the endoscope. Such a center line of the working channel may extend in parallel to a center line of the insertion tube.

The endoscopes of the present disclosure, or one or more parts thereof, such as the insertion tube and/or a distal end thereof and/or a tip part assembly thereof, may be suitable for or adapted for insertion into a body cavity, such as a kidney, through a body opening, such as a urinary passage or a urethra for visually inspecting places that are difficult to access, such as human body cavities. The body may be a natural and/or artificial body, potentially a human body. The insertion tube may extend from a or the operating handle towards a distal end of the endoscope.

The endoscopes of the present disclosure may be single-use or disposable endoscopes and/or may be repeated-use endoscopes. The endoscopes may comprise an elongate insertion tube, potentially with an operating handle at a proximal end, as seen from the operator, and visual inspection means, such as a built-in camera, at a distal end of the insertion tube. The endoscopes may be equipped with a light source and/or a vision receptor including a vision sensor, such as a camera or an image sensor, one or more of which may be positioned in a tip part assembly of the endoscope. With the endoscopes, it may be possible for the operator to see where the endoscope is steered and to set the target of interest once a tip of the endoscope has been advanced thereto. Electrical wiring, potentially for the camera and/or other electronics, may run along an inside of the insertion tube, potentially from an operating handle to a tip part assembly of the endoscope. In cases where the working channel is used for suction, the working channel can be denoted "a suction channel", and the working channel tube a "suction channel tube". The suction channel may be connected to a suction connector, typically positioned at a handle at the proximal end of the insertion tube. The working channel may additionally, or alternatively, be for air flow into or out of the body cavity and/or administration of pharmaceuticals. The endoscopes of the present disclosure may further comprise a proximal operating handle and/or a distal tip part assembly. A proximal end of the insertion tube may be connected to the operating handle. The tip part assembly may be connected to a distal end of the insertion tube. The insertion tube may connect the handle to the tip part assembly. The working channel tube may extend from the handle, though the insertion tube, and to and/or through the tip part assembly, potentially to a distal port of the working channel. The operating handle may be suitable for allowing an operator to grip and to operate the endoscope, potentially with one hand. The operating handle may comprise a handle housing arranged at a proximal end of the insertion tube. The operating handle may be directly or indirectly connected to the insertion tube. In the latter case, one or more connection parts may be provided in between.

Generally, in the endoscopes of the present disclosure, a distal end of a tip part assembly of the endoscope may be positioned oppositely from the proximal end and may form a distal end of the endoscope. The tip part assembly can include a housing having a circumferentially extending outer surface for facing the environment. The outer surface may enclose a volume and may extend in a longitudinal direction between a proximal end and a distal end of the housing. The working channel or a distal part or end thereof may be at least partly housed in the housing and may comprise an opening in a distal surface of the housing. The camera assembly may be partly or entirely housed in the housing. The housing may be an outer or exterior housing, potentially for facing the outer sleeve and/or the environment, which may be exterior with respect to components or elements housed or enclosed therein, such as a camera module, an FPC, wires, electrical components, LEDs, and the like.

Generally, in the endoscopes of the present disclosure, a housing of a tip part assembly may be connected to a distal end segment of the bending section. The housing may be connected to the distal end segment at the proximal end of the housing. The working channel may extend into and/or through the distal end segment and/or the bending section. A part of the working channel not being the working channel tube may be integral and/or in one piece with the housing.

Generally, in the endoscopes of the present disclosure, a tip part assembly of the endoscopes of the present disclosure may be directly or indirectly connected to the insertion tube. In the latter case, one or more connection parts may be provided in between.

The endoscopes disclosed herein may comprise a control element. The control element may be configured to allow an operator to control or bend a distal part of the endoscope, potentially by activation of at least one steering wire. The control element may allow bending a tip part assembly in at least one direction, potentially in two directions, the two directions potentially being opposite. The control element may be accommodated in an operating handle. The control element may include a lever allowing an operator to control the control element. The lever may extend outwardly from the control element, potentially through an or the operating handle. The control element may be in the form of a roller or a roller disc.

To be able to maneuver the endoscopes of the present disclosure inside a body cavity, a distal end of the endoscopes of the present disclosure may comprise a bending section with increased flexibility, e.g. an articulated tip part assembly allowing the operator to bend this section. Such a bending section may connect a distal part or end of the insertion tube to a tip part assembly of the endoscope. This may be achieved by tensioning or slacking steering wires, which may run along an inside of the elongated insertion tube, potentially outside the working channel and/or working channel tube, potentially from the bending section to a control mechanism of an operating handle of the endoscope. The working channel and/or working channel tube may extend through such a bending section. Steering wires may extend outside the working channel and/or bending section. The bending section which may have a distal end segment which may be connected to a housing of the tip part assembly. The bending section may comprise a number of hingedly interconnected segments including a distal end segment, a proximal end segment, and a plurality of intermediate segments positioned between the proximal end segment and the distal end segment. At least one hinge member may interconnect adjacent segments with each other. The bending section may be a section allowing a distal portion of the endoscope to bend, potentially so as to allow an operator to manipulate the tip part assembly while inserted into a body cavity of a patient. The bending section may be molded in one piece or may be constituted by a plurality of molded pieces.

A potting material, such as an adhesive, may be provided in the housing. The potting material may fill out any or some parts of a spacing between components inside the housing, e.g. the working channel and/or working channel tube, a camera module, and/or an FPC, or parts of the latter components arranged inside the housing.

Generally, in the endoscopes of the present disclosure, a connector and/or a connecting portion may be provided at a proximal end of the endoscope to allow insertion of a tool into the working channel and/or to allow suction to be applied to the working channel. In an embodiment, the working channel comprises a built-in or integrated tool at or in the distal tip part assembly. Such a tool may be suitable for grabbing, taking, and/or holding elements in a part of a patient, in which the endoscope tip part is arranged during use.

A camera assembly of the endoscopes of the present disclosure may be positioned in or partly in a tip part assembly of the endoscope. Such a camera assembly may be positioned separately and/or outside the working channel tube. Such a camera assembly may comprise a vision sensor and/or a lens stack and/or a camera housing.

In an embodiment, the endoscope or a tip part assembly thereof comprises one or more light sources, such as LEDs, and/or one or more light guides for guiding light from respective LED(s) to e.g. a front or distal end surface or end wall of the endoscope or tip part assembly. Such light sources and/or light guides may be positioned within the endoscope and/or a tip part assembly thereof and may be positioned outside the working channel and/or working channel tube. Alternatively, one or more optical fibers can extend from the operating handle to a light guide in the tip part, the light source being positioned e.g. in the operating handle, a display unit, or being provided separately from the endoscope.

Generally, in the endoscopes of the present disclosure, an exterior flexible sleeve may be provided to extend around at least part of the endoscope and/or a tip part assembly thereof and/or the insertion tube thereof. The insertion tube and/or working channel and/or working channel tube and/or a bending section of the endoscope may be positioned inside the flexible sleeve. The flexible sleeve may form at least part of an exterior surface of the endoscope for facing the environment.

The working channel tube of the endoscopes described herein may be flexible. The working channel tube may at the tip part assembly be held in a potentially circumferential working channel tube holder. Such a working channel tube holder may similarly be tubular and may have an internal diameter that corresponds to an outside diameter of the working channel tube, a distal end of the working channel tube being positioned and held in the working channel tube holder. The working channel tube holder may be substantially rigid and/or may be formed integral with or in one piece with a housing of a tip part assembly of the endoscope. Such a tube holder may be combined with a distal portion of the working channel as described above, the working channel tube being inserted into the tube holder. The working channel tube may be provided as a part separate from one or more or all other components, parts, or elements of the endoscope and/or of a tip part assembly and/or an operating handle thereof. Such components, parts, or elements of the endoscope may include any one or more of all components, parts, or elements of endoscopes as described herein.

Generally, in the endoscopes of the present disclosure, the working channel tube may include a circumferential tube wall enclosing a working channel spacing. In an embodiment, no such tube holder is present, the working channel tube potentially not being held in the tip part assembly or being held in the tip part assembly in another way. In the latter case, a distal end of the working channel tube may be held by being inserted into a distal opening of the endoscope and/or of a tip part assembly thereof, potentially a housing of the latter. In other embodiments, a distal end of the working channel tube extends only to a circumferential or tubular part, the latter part then extending from the distal end of the working channel tube to a distal opening of the tip part assembly or the endoscope, this circumferential or tubular part thus providing a distal part of the working channel, the working channel tube providing the remaining proximal part of the working channel. In the latter embodiments, the working channel tube may similarly be inserted into the circumferential or tubular part, or the working channel tube distal end may be positioned to abut a proximal end of the circumferential or tubular part. Such a circumferential or tubular part may in the latter case comprise a circumferential wall, which may be of substantially same diameter as that of the working channel tube.

The endoscopes of the present disclosure may be of a type, which does not comprise an everting driving tube means and/or in which the working channel tube is not for contacting any part of a patient during use or operation of the endoscope and/or in which the insertion tube, potentially in any use position of the endoscope, shields or protects all parts of the working channel tube from the surroundings. The working channel tube may be non-evertible or non-everted or non-everting, which may involve that the working channel tube is destroyed or broken if it is attempted to evert it or if it is everted. This may be achieved by the working channel tube or materials of the working channel tube having a high rigidity or hardness as also described elsewhere in the present disclosure.

Generally, in the endoscopes of the present disclosure, one or more light sources, such as LEDs, may be positioned outside the working channel and/or working channel tube, and potentially inside a tip part assembly of the endoscope.

Generally, in the endoscopes of the present disclosure, the working channel tube and working channel spacing may extend from the handle of the endoscope to the tip part assembly, potentially all the way to a distal end of the tip part assembly and/or of the endoscope. The working channel spacing can be considered a channel in itself. A proximal opening or port of the working channel tube may provide access to insertion of a surgical instrument into the working channel spacing or may provide connection to suction means. A distal opening or port of the working channel tube and/or the endoscope, potentially at or in the tip part assembly, may allow exit of the tool or surgical instrument from the working channel spacing or may, respectively, provide suction into the working channel spacing. Hereby, the working channel tube can guide the tool or surgical instrument or the like through the insertion tube and into and out of a body cavity, potentially so that an external surface of the surgical instrument or the like, potentially including a tip thereof, can glide or slide on an inner surface of the working channel tube. Similarly, the working channel tube can provide suction from a body cavity through the insertion tube so that fluids and/or bodily tissue can be sucked out of a body cavity, through the insertion tube and out of the endoscope, potentially to be collected outside the endoscope.

In an embodiment, no parts and/or elements and/or components of the endoscopes as disclosed herein are positioned within the working channel and/or working channel tube and/or working channel spacing. Any one or more of the parts and/or elements and/or components of the endoscopes as disclosed herein may be positioned outside the working channel and/or working channel tube and/or working channel spacing. An insertion tool or parts thereof, and/or bodily fluids and/or tissue, and/or sensors, one or more motion transfer members or the like, may be positioned in the working channel and/or working channel tube and/or working channel spacing.

Generally, in the endoscopes of the present disclosure and methods of manufacture thereof, the working channel tube may exist without the other parts of the endoscope, and the applicant reserves the right to claim such working channel tubes and methods isolated from the endoscope, i.e. in and by themselves and not comprising other parts of the endoscope.

In an embodiment of the endoscopes of the present disclosure, the outer layer has been extruded or extrusion coated on the inner layer or on a coextrusion comprising the inner layer so that the outer layer is attached to the inner layer or to the coextrusion comprising the inner layer.

Potentially, no further layer(s) is (are) provided between the inner layer or a coextrusion comprising the inner layer and the outer layer or a coextrusion comprising the outer layer. Alternatively, one or more further layers, such as an adhesive or glue layer, may be provided between the inner layer or a coextrusion comprising the inner layer and the outer layer or a coextrusion comprising the outer layer.

In an alternative embodiment, the inner and outer layers have been coextruded with each other together with zero, one, or more intermediate layers, wherein one or more of the intermediate layers is optionally a coextrusion tie layer.

In an embodiment, the endoscope further comprises a proximal operating handle and a distal tip part assembly, wherein a proximal end of the insertion tube is connected to the operating handle, and the tip part assembly is connected to a distal end of the insertion tube, so that the insertion tube connects the handle to the tip part assembly, and wherein the working channel tube extends from the handle, though the insertion tube, and to and/or through the tip part assembly.

In another embodiment, a total number of extruded or coextruded layers of the working channel tube is two. This total number of coextruded layers can be more than two, such as three, four, five, or six.

In another embodiment, at a proximal end of the working channel tube, a proximal portion of the working channel tube is everted and positioned in an assembly part accommodating the everted tube portion.

In another embodiment, a wall thickness of the inner layer is within a range of 0.01 to 1 mm, and a wall thickness of the outer layer is within a range of 0.1 to 0.5 mm.

In embodiments in which the working channel tube has an inner diameter of 2 to 3.5 mm, the wall thickness of the inner layer may be within a range of 0.01 to 1 mm, and a wall thickness of the outer layer may be within a range of 0.1 to 0.3 mm, such as 0.2 mm.

In embodiments in which the working channel tube has an inner diameter of 3.5 to 5 mm, the wall thickness of the inner layer may be within a range of 0.01 to 1 mm, and a wall thickness of the outer layer may be within a range of 0.3 to 0.5 mm, such as 0.4 mm.

In another embodiment, the working channel tube is a one-piece tube.

The working channel tube may consist of one single piece of tube and/or may or may not be assembled from several pieces of tube. The working channel tube may alternatively or additionally be an integral or unitary tube and/or piece of tube.

In another embodiment, the working channel tube extends through an entire length of the insertion tube and/or through a bending section of the endoscope and/or through a tip part assembly of the endoscope.

In another embodiment, the working channel tube has a circumferentially extending, substantially cylindrical outer wall enclosing an inner working channel spacing.

In another embodiment, a wall thickness of the coextruded layers of the working channel tube is substantially the same along an entire length of the working channel tube.

In another embodiment, the working channel tube comprises a coil or spring positioned outside and/or inside and/or between extruded and/or coextruded layers of the working channel tube, for example between an extruded outer monolayer and an extruded inner monolayer or between an outer monolayer and an inner multi-layer coextrusion comprising the inner layer.

Alternatively, or additionally, the coil or spring is positioned outside and/or inside one or more layers of the working channel tube and or is positioned between two layers of the working channel tube, wherein one or both these layers may be extruded. Such a coil may prevent kink of the working channel tube. The coil may extend along an entire length of or only along one or more portions of the working channel tube. The coil may be a helical coil or a helical spring. The coil may be positioned in a correspondingly shaped track in one or more surfaces of layers of the working channel tube. Such a track may be cut into a surface of the associated layer. The coil may be of a metal or polymer or plastic material. The coil may be replaced by another suitable type of support member suitable for preventing kink of the working channel tube.

The coil may be positioned between the outer layer and the inner layer. This may be achieved by first extruding the inner layer as a monolayer or as forming part of a coextrusion. Then, the coil can be positioned around the inner layer or the coextrusion comprising the inner layer. Positioning of the coil may be done immediately after the manufacture of the inner layer or of the coextrusion comprising the inner layer, potentially so that the manufacture of the inner layer or the coextrusion comprising the inner layer and the positioning of the coil occurs in a continuous process, potentially to produce and endless tube including the coil, which endless tube can later be cut into individual working channel tubes for endoscopes. Alternatively, the coil can be positioned around the inner layer or the coextrusion comprising the inner layer after the inner layer or the coextrusion comprising the inner layer has been cut to individual pieces. When the coil has been suitably positioned, the outer layer, which can be a monolayer or be included in another coextrusion, is extruded or coated or coextrusion coated on the inner layer or on the coextrusion comprising the inner layer. Besides the inner layer, a coextrusion comprising the inner layer may, for example, comprise a coextrusion tie (sub-)layer tying the inner layer to a third (sub-)layer of the coextrusion. Similarly, besides the outer layer, a coextrusion comprising the outer layer may, for example, comprise a coextrusion tie (sub-)layer tying the inner layer to a third (sub-)layer of the coextrusion comprising the outer layer.

In an embodiment, the inner layer is a sub-layer of a coextrusion comprising at least one further sub-layer, and the outer layer is extruded or extrusion coated on the coextrusion, potentially on a contact layer of the coextrusion, the coextrusion also comprising the inner layer.

In a development of the latter two embodiments, the inner layer forms part of a coextrusion comprising the inner layer as a sub-layer and at least one further sub-layer, and the outer layer is a mono layer extruded or extrusion coated on the coextrusion. In this case, for example, the coil may be positioned between the outer layer and the inner layer. This may be achieved by first extruding the coextrusion comprising the inner layer. Then, the coil is positioned around the coextrusion comprising the inner layer, i.e. around the third sub-layer or contact layer of the coextrusion. When the coil has been suitably positioned, the outer layer in the form of a monolayer is extruded or extrusion coated on an outer surface of the third sub-layer or contact layer of the coextrusion comprising the inner layer.

A sub-layer of the coextrusion including the inner layer may be a contact layer that contacts the outer layer. This contact layer may have one or more of the characteristics of the outer layer and may be of a material corresponding to the characteristics of or that is identical to the outer layer material as disclosed herein. Hereby, the contact layer can have a relatively high Shore hardness, which can have the effect that the coil will lead to less bulging-out of the inner layer, such bulging-out being caused by the coil. At the same time, the inner HDPE layer can keep the surface friction of the inside surface of the inner layer relatively low.

In another aspect, the present disclosure relates to a method of manufacture of any one of the endoscopes as disclosed herein, the method comprising
extruding or coextruding the inner and outer layers of the working channel tube to provide the working channel tube; and
inserting the working channel tube into the insertion tube so that the working channel tube establishes at least part of the working channel of the endoscope.

In this and other aspects, extruding the inner and outer layers of the working channel tube may involve first extruding the inner layer and then extruding or extrusion coating the outer layer on the inner layer or on a coextrusion comprising the inner layer so that the outer layer is attached to the inner layer or to the coextrusion comprising the inner layer.

Extruding the inner and outer layers of the working channel tube may alternatively involve coextruding the inner and outer layers with each other together with zero, one, or more intermediate layers, wherein one or more of the intermediate layers is optionally a coextrusion tie layer.

In another aspect, the present disclosure relates to a method of manufacture of an endoscope,
the endoscope comprising a working channel extending through an elongate insertion tube to allow for insertion of a retractable instrument into a body and/or for suction of fluids from the body through the working channel,
wherein the method comprises:
extruding an inner layer material and an outer layer material to form a working channel tube of the endoscope, so that an inner layer of the working channel tube comprises the inner layer material and an outer layer of the working channel tube comprises the outer layer material,; and
inserting the working channel tube into the insertion tube so that the working channel tube establishes at least part of the working channel of the endoscope.

The inner layer material and the outer layer material may be according to or have one or more of the properties of any one or more of the examples of the inner layer material and outer layer material, respectively, as disclosed herein. For example, the outer layer material can be a thermoplastic polyurethane having a Shore A hardness according to ASTM D2240-15 within a range of 85 to 100, and the inner layer material can be a high density polyethylene

In the present and other aspects, extruding the inner layer material and the outer layer material of the working channel tube may involve first extruding the inner layer to form the inner layer and then extruding or extrusion coating the outer layer on the inner layer or on a coextrusion comprising the inner layer so that the outer layer is attached to the inner layer or to the coextrusion comprising the inner layer.

Extruding the inner layer material and the outer layer material of the working channel tube may alternatively involve coextruding the inner layer material and the outer layer material with each other together with zero, one, or more intermediate layers, wherein one or more of the intermediate layers is optionally a coextrusion tie layer.

In this and other aspects of the present disclosure, at a proximal part of the working channel tube, an outer surface of the outer layer can be glued or adhered to the operating handle, and/or at a distal part of the working channel tube, an outer surface of the outer layer can be glued or adhered to the housing of the tip part assembly.

In an embodiment of the methods of manufacture as disclosed herein, the working channel tube is cut from a tube or an endless tube having the structure or cross-section of the working channel tube. The working channel tube may thus be cut from an endless tube and may be cut to a desired length, potentially in a length as disclosed elsewhere herein. The cutting step may occur before, during, or after assembly of the endoscope, i.e. of the components of the endoscope as disclosed and described herein.

In the methods of manufacture of endoscopes according to the present disclosure, the working channel and working channel tube may be and/or may be manufactured according to the working channels and working channel tubes as disclosed herein.

In the methods of manufacture of endoscopes according to the present disclosure, the manufactured endoscope may be according to any one of the embodiments of endoscopes as disclosed herein.

The methods of this disclosure may generally provide identical or similar advantages to the endoscopes according to the first aspect of this disclosure and as described herein. Embodiments of working channel tubes of these methods or any other structural features or elements of these methods may be the same as described with respect to the endoscopes.

In another aspect, the present disclosure relates to a system for visually inspecting inaccessible places such as human body cavities, the system comprising an endoscope according to any one of the embodiments of endoscopes as disclosed herein and a monitor or display.

The monitor or display may be for displaying an image provided by a vision sensor or camera of the endoscope. The monitor may include a display.

The endoscope may be connectable or connected to the monitor or display, and/or the monitor or display may allow an operator to view an image captured by the vision sensor of the endoscope.

A person skilled in the art will appreciate that any one or more of the above aspects of this disclosure and embodiments thereof may be combined with any one or more of the other aspects of the disclosure and embodiments thereof.

### Brief description of drawings

The tip part assemblies and methods will now be described in greater detail based on non-limiting exemplary embodiments and with reference to the schematic drawings, on which:
FIG. 1a is a perspective view of an embodiment of the endoscopes according to the present disclosure;
FIG. 1b is a perspective view of a monitor to which the endoscope of FIG. 1a can be connected;
FIG. 2a is a cross-sectional view through a first embodiment of the working channel tubes according to the present disclosure as implemented in the endoscope of FIG. 1a;
FIG. 2b is a cross-sectional view through a second embodiment of the working channel tubes according to the present disclosure as implemented in the endoscope of FIG. 1a;
FIG. 3a is a side view of a third embodiment of the working channel tubes according to the present disclosure as implemented in the endoscope of FIG. 1a;
FIG. 3b shows a detail B in FIG. 3a of a cross-sectional view of the working channel tube of FIG. 3a, taken along the line A-A of FIG. 3a;
FIG. 4a is a distal end plane view of a tip part assembly of the endoscope of FIGs 1a and 1b;
FIG. 4b is a cross-sectional view of the tip part assembly of FIG 4a taken along the line D-D of FIG. 4c;
FIG. 4c is another cross-sectional view of the tip part assembly of FIG. 4a, taken orthogonally to the cross-sectional view of FIG. 4b and along the line C-C of FIG. 4a;
FIG. 5 is a perspective view of the working channel tube of FIG. 2a inserted into the tip part assembly of FIGs 4a to 4c; and
FIG. 6 is a cross-sectional view of a proximal end of the working channel tube of FIG. 2a, the working channel tube having been everted at its proximal end.

### Detailed description of embodiments

Similar reference numerals are used for similar elements across the various embodiments and figures described herein.

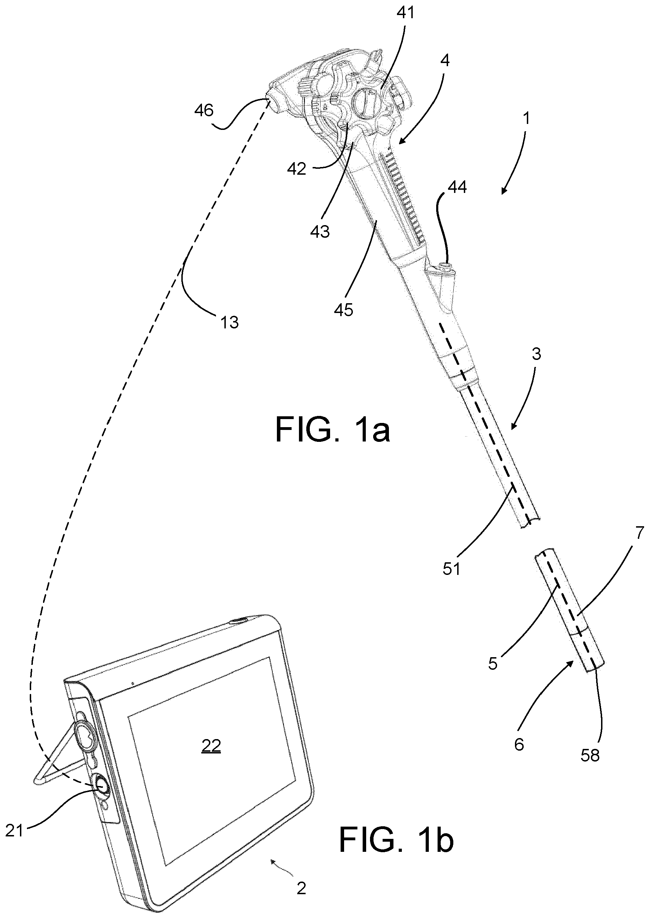

FIG. 1a shows an endoscope 1 according to an embodiment of the endoscopes of the present disclosure. The endoscope can be any type of endoscope, such as a gastroscope, a duodenoscope, a bronchoscope, a rhinolaryngoscope, or a cystoscope.

The endoscope 1 is or can be connected to a monitor 2 shown in FIG. 1b. The monitor 2 is an image processing unit capable of displaying images captured by the endoscope 1 on a screen, which may be a part of the monitor 2 or a separate device connected to the monitor 2. The endoscope 1 is disposable and single-use, i.e. not intended to be cleaned and reused. The endoscope 1 comprises an elongated insertion tube 3. At a proximal end of the insertion tube 3, an operating handle 4 is arranged. The operating handle 4 has a control lever 41, which is known *per se* and comprises two control knobs 42, 43, respectively, for maneuvering a tip part assembly 6 attached at a distal end of the insertion tube 3 by means of one or more not shown steering wires. In a known operation of the control lever 41, each knob 42, 43 is operated by one or more fingers of an operator to control a bending operation of a tip of the endoscope 1, the tip for this purpose comprising a not shown and known bending section which allows bending in two dimensions, each direction corresponding to the operation of one of the two knobs 42, 43. The control lever 41 can comprise a known locking or arresting mechanism, upon activation of which a selected bending configuration of the tip can be maintained in a known manner. Other known types of control systems could alternatively be implemented in the endoscopes of the present disclosure.

The endoscope 1 comprises a working channel 5 comprising a working channel tube 51. The working channel tube 51 is shown schematically with a dashed line in FIG. 1a. The working channel 5 extends through the insertion tube 3 to allow for insertion of a retractable instrument or tool into a not shown body and/or for suction of fluids from the body through the working channel 5. The working channel 5 extends from a distal end thereof, which end is secured at and/or forms part of the distal end of the endoscope 1, in a longitudinal direction and, at a proximal end thereof, is secured to the operating handle 4. The working channel 5 extends from a distal end of the endoscope 1 where it has an orifice or exit port 58 at or of the tip part assembly 6 to an entry port 44 at the operating handle 4. A surgical tool may be introduced into the working channel 5 via the entry port 44 and advanced through the working channel 5 to extend out of the exit port 58 at the tip part assembly 6.

The working channel tube 51 is proximally secured at and to the operating handle 4 and the entry port 44 by means of application of a glue or adhesive between a part of the handle 4 and the outer layer 53 to secure them to each other. Distally, the working channel tube 51 can similarly be secured to a housing 62 of a tip part assembly 6 by means of application of a glue between the two parts to secure them to each other. The working channel tube 51 may not be secured to other parts of the endoscope 1 in any other positions of the tube 51. The glue is in contact with and adheres to an outer surface of the outer layer 52 of the working channel tube 1 to secure the working channel tube 1.

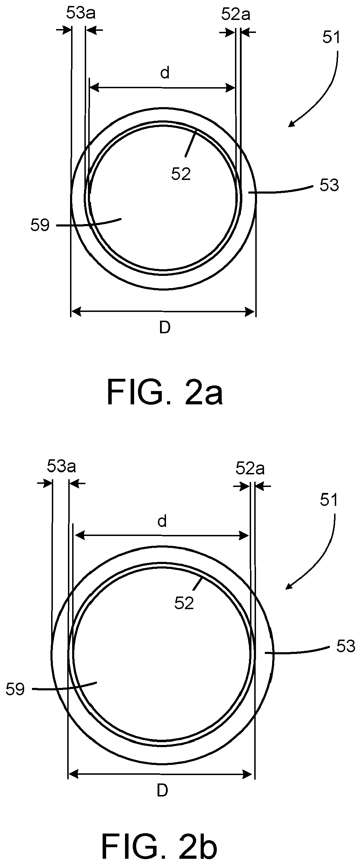

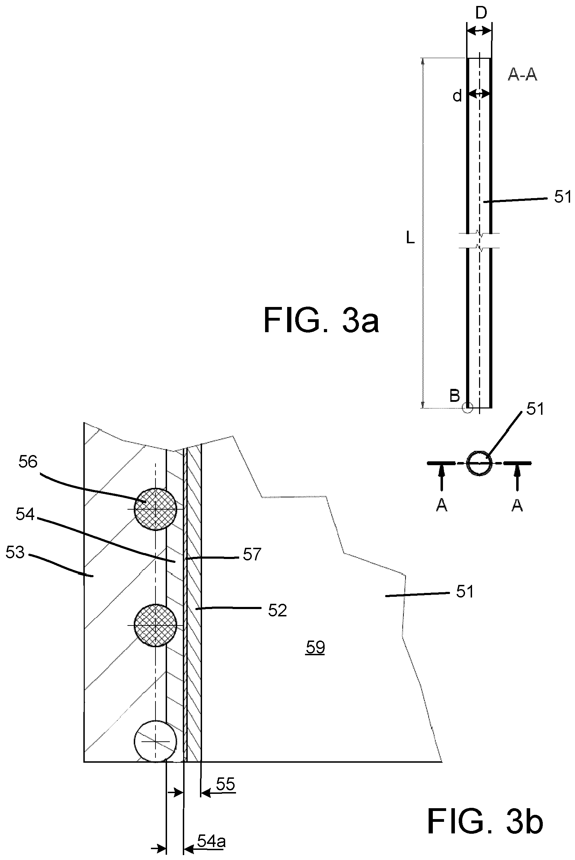

Three different embodiments of the working channel tube 51 of FIG. 1a are shown in FIGs 2a, 2b, and 3a, 3b, respectively. In all three embodiments, the outer layer 53 is a monolayer. In the embodiments of FIGs 2a and 2b, the inner layer is also a monolayer. In the embodiment of FIGs 3a and 3b, the inner layer 54 is a sub-layer of a coextrusion.

In all three embodiments, the working channel tube 51 can be cut from an initially manufactured longer piece of tube, potentially an endless tube, and comprises the inner layer 52 of an inner layer material and the outer layer 53 of an outer layer material. The working channel tube 51 of all three embodiments is substantially tubular and has a circumferentially extending, substantially cylindrical or circular cylindrical, outer wall enclosing an inner working channel spacing 59. In all three embodiments, the outer layer material is a thermoplastic PU having a Shore A hardness according to ASTM D2240-15 within a range of 85 to 100, and the inner layer material is a high-density polyethylene. The inner layer 52 is in all three embodiments of an HDPE with a melt index of 0.2 to 0.4 g/10 min according to ISO 1133, a flexural modulus of 1300 to 1500 MPa according to ISO 178, a tensile modulus of 1200 to 1400 according to ISO 527-2, a tensile strain at yield (50 mm/min) of 6 to 10 % according to ISO 527-2, and a tensile strength at yield (50 mm/min) of 27 to 32 MPa according to ISO 527-2. The HDPE is an extrudable and coextrudable and bimodal HDPE.

In all the three embodiments, the inner and outer layers 52, 53 as well as the working channel tube 51 generally have substantially unchanged associated wall thicknesses along an entire length of the working channel tube 51.

In all three embodiments, the Shore A hardness of the or of the material of the outer layer 53 is about 90. The Shore D hardness of the or of the material of the inner layer 52 is about 63. The inner and outer layers 52, 53 can comprise barium sulphate (BaSO₄), which is particularly relevant for the outer layer 53.

In all three embodiments, the PU of the outer layer 53 is an extrudable PU. In the embodiments of FIGs 2a and 2b, the PU is a thermoplastic PU elastomer having a specific gravity according to ASTM D 792 of 1.1 to 1.2, a Tensile modulus according to ASTM D 412 of 6 to 7 MPa (50 % elongation) and/or of 9 to 11 MPa (100 % elongation) and/or of 18 to 20 MPa (300 % elongation), an ultimate tensile strength according to ASTM D412 of 35 to 45 MPa, an ultimate elongation according to ASTM D412 of 500 to 600 %, an elongation set after break according to ASTM D412 of 50 to 70 %, a tear strength (Die C) according to ASTM D624 of 90 to 100 MPa, compression set according to ASTM D 395 (Method B) of 20 to 30 % (25°C) and/or 35 to 45 % (70°C), A Taber abrasion resistance according to ASTM D 1044 (1000 g, 1000 cycles, H-22 wheel (coarser)) of 40 to 70 mg, a flexural modulus according to ASTM D 790 of 65 to 75 MPa, a vicat softening temperature according to ASTM D 1525 of 85 to 95°C, a coefficient of linear thermal expansion according to ASTM D 696 of 150 to 170 10⁻⁶ mm/mm/°C, a glass transition temperature according to DSC of -30 to -36°C, and a melt index according to ASTM D 1238 (224°C, 1.2 kg load) of 30 to 35 g/10 m. The Shore A hardness of this material is about 87.

In the embodiment of FIGs 2a and 2b, the inner and outer layers 52, 53 have been coextruded with each other. No intermediate layers, such as a coextrusion tie layer, are provided between the inner and outer layers. In other embodiments, one or more intermediate layers, such as one or more coextrusion tie layers, can be provided between the inner and outer layers 52, 53, potentially to improve adhesion between the inner and outer layers 52, 53. In the embodiment of FIG. 2a, the wall thickness 52a, i.e. the layer thickness, of the inner layer 52 is about 0.05 mm. The wall thickness 53a, i.e. the layer thickness, of the outer layer 53 is about 0.25 mm. The inner diameter d of the working channel tube 51 is about 2.2 mm and the outer diameter D is about 2.7 mm.

In the embodiment of FIG. 2b, the inner diameter d is about 2.8 mm, the outer diameter D about 3.3 mm, and the wall thicknesses 52a, 52b are the same as in the embodiment of FIG. 2a.

In a similar, not shown working channel tube with an inner diameter of 4.3 mm and an outer diameter of 5.2 mm, the inner layer 52 has a thickness of 0.05 mm and the outer layer 53 has a thickness of 0.4 mm.

The embodiment of FIGs 3a and 3b includes an inner layer 52 and an outer layer 53. The outer layer 53 is a mono layer. The inner layer forms part of a coextrusion which also includes an intermediate coextrusion tie (sub-)layer and a contact (sub-)layer 57. The inner layer 52 has, thus, first been coextruded together with the tie layer 57 and the contact layer 54. Then, a coil 56 has been positioned around the layer 54, and then the outer layer 53 has been extruded or extrusion coated on an outer surface of the contact layer 54. The coil 56 is a stainless steel 304V spring having a wire thickness or diameter of 0.152 mm and a WPI (Wraps Per Inch) of 60.

The inner layer 52 thus forms part of a coextrusion comprising the inner layer 52 as a sub-layer and two further sub-layers 54, 57. The outer layer 53 has been extruded or extrusion coated on the coextrusion to form an extrusion or extrusion coating on the contact layer 54. The coil 56 is positioned between the outer layer 53 and the (contact layer 54 of the) coextrusion.

This is achieved by first coextruding the coextrusion comprising the layers 52, 54, 57. Then, the coil 56 is positioned around the coextrusion comprising the layers 52, 57, 54, i.e. around the contact layer 54. This can be done immediately after the coextrusion process, potentially so that the coextrusion and the positioning of the coil 56 occurs in a continuous process. Alternatively, the coil 56 can be positioned around the contact layer 54 after the coextrusion has been cut to individual pieces.

When the coil 56 has been suitably positioned, the outer layer is extruded or extrusion coated on an outer surface of the contact layer 54. Hereby, the parts of the coil 56 not embedded in the track of the contact layer 54 are embedded in the outer layer material as shown in FIG. 3b. About 0.114 mm of the coil wire diameter is embedded in the outer layer 53.

As schematically shown in FIG. 3b, due to spring forces of and/or push forces of the outer layer 53, the coil 56 is pushed inwardly by the outer layer 53 so as to deform the contact layer 54 to provide a helical track in the layer 54 corresponding to the helical shape of the coil 56. This helical track can alternatively be cut into the outer surface of the contact layer 54 before the coil 56 is positioned in the track to surround the coextrusion. In an alternative embodiment, the coil 56 can instead be positioned on an outside of the outer layer 53 to extend around the outer layer 53, potentially wholly or partly embedded in an outer, similar track, potentially cut or formed-by-deformation, of an outer surface of the outer layer 53.

In the embodiment shown, a track depth of the track in the layer 54, in which the coil 56 is embedded, is about 0.038 m.

The layers 53, 54 are of an aliphatic polyether-based thermoplastic polyurethane having a specific gravity according to ASTM D792 of 1.06 to 1,10 g/cc, a linear mold shrinkage according to ASTM D955 of 0.0050 to 0.0012 cm/cm, a hardness shore A of 85 to 89, a tensile strength, ultimate, according to ASTM D412 of 50 to 55 MPa, an elongation at break according to ASTM D412 of 380 to 400 %, a 100% modulus according to ASTM D412 of 6.8 to 7 MPa (at strain 100 %), a 200% modulus according to ASTM D412 of 12 to 14 MPa (at strain 200 %), a 300% modulus according to ASTM D412 of 28 to 31 MPa (at strain 300 %) and a flexural modulus according to ASTM D790 of 0.02 to 0.05 MPa.

Thus, the material of the contact layer 54 has the same characteristics as the material of the outer layer 53, meaning that the contact layer 54 will also have a similar, high Shore A hardness. This can have the effect that the coil 56 will lead to less inwards bulging-out of the inside layer 52, this bulging-out being caused by the coil 56. At the same time, the inside layer of HDPE can keep the surface friction of the inside surface of the inner layer relatively low.

The embodiments of FIGs 2a and 2b includes no such coil or spring 56; however, a coil or spring could be positioned between the inner and outer layers 52, 53. In this case, the working channel tube 51 could be manufactured in a manner similar to described above for the embodiment of FIGs 3a and 3b. Thus, the inner layer 52 could be extruded first as a monolayer, the coil 56 could then be positioned around the inner layer 52, potentially in a cut track thereof, and the outer layer 53 could then be extruded on an outer surface of the inner layer 52 and on the coil 56 so that coil 56 would be embedded in the outer layer 56.

In the embodiment of FIGs 3a, 3b, the total wall thickness 55 of the inner layer 52 and a tie layer 57 positioned between the inner and outer layers 52, 53 is about 0.0635 mm. The tie layer 56 wall thickness is thin and is less than 0.0127 mm. The wall thickness 54a of the layer 54 is also about 0.635 mm. The inner diameter d of the working channel tube 51 is about 4.3 mm and the outer diameter D is about 5.15 mm.

The coextrusion tie layer 57 is a maleic anhydride grafted linear low-density polyethylene and has a melt index according to ISO 1133/ASTM D1238 of 2 to 2.5 g/10 min, a melting point according to ISO 11357-3 of 110 to 130°C and/or a Vicat softening temperature (10N; on compression molded samples) according to ISO 306/ASTM D1525 of 80 to 90°, and a density according to ISO 1183/ASTM D1505 of 0.90 to 0.92 g/m³. Such a tie layer 57 can improve adherence between the inner layer 52 and the contact layer 54 in the coextrusion.

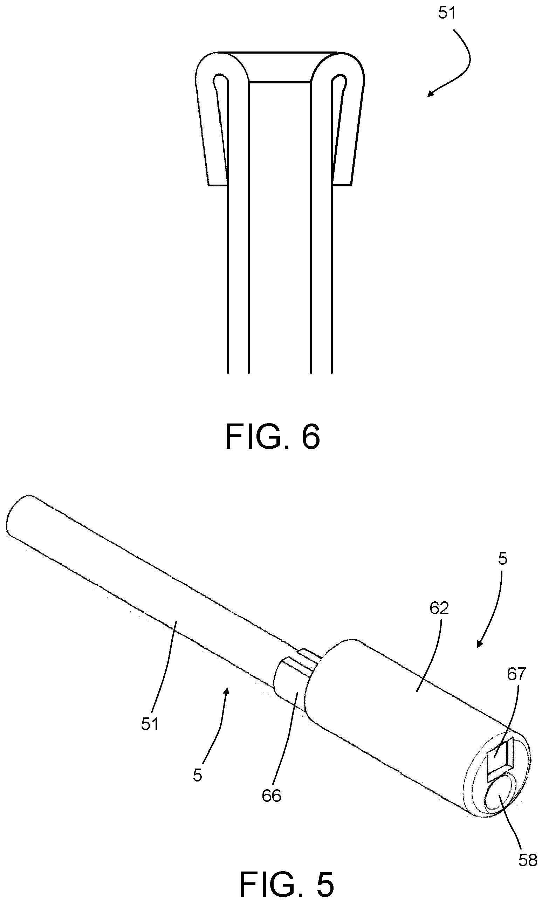

FIG. 6 schematically shows a proximal end or part of the working channel tube 51 of any one the embodiments of FIGs 2a and 2b everted so that it may be positioned in a not shown assembly part accommodating the everted tube portion. A few mm of the proximal part of the working channel tube 51 is thus everted or folded upon itself. This may also be done with the working channel tube 51 of FIGs 3a, 3b and can reduce or remove a risk of delamination of the inner and outer layers 52, 53. Thus, to ensure that movement of a tool through the working channel tube 51 is not hindered if the layers 52, 53 should delaminate from each other, at a proximal end of the endoscope 1 shown in FIG. 1a, adjacent the working channel opening or entry port 44 of the operating handle 4, a proximal portion of the working channel tube 51 can be everted and positioned in an assembly part accommodating the everted tube portion. As shown in FIG. 1a, the entry port 44 to the working channel 5 includes a connector or pipe connection or branch piece connected to the working channel tube 51 in a not shown manner. Due to the presence of the tie layer 57 in the working channel tube 51 of FIGs 3a, 3b, when coextruded, the inner layer 52 and the contact layer 54 bond so well to each other that delamination of the inner and outer layers 52, 53 does not occur during normal use. To reduce a risk of delamination, an extrusion primer may be added to the outer surface of the inner layer 52 of the embodiment of FIGs 2a and 2b or to the outer surface of the contact layer 54 of the embodiment of FIGs 3a and 3b to improve adherence to the outer layer 53.

The working channel tube of FIGs 2a and 2b consists only of the two coextruded layers 52, 53, whereas that of FIGs 3a, 3b consists of the layers 52, 53, 54, 57 and the coil 56 provided in and between the layers 53, 54. The working channel tube 51 of all three embodiments does not comprise any non-extruded layers, such as coatings and/or surface treatments.

The working channel tube 51 of all three embodiments is flexible.

In the embodiment shown in FIG. 1a, the working channel tube 1 is the only working channel tube 1 of the endoscope 1 and it, as well as each of the inner and outer layers 52, 53, each extends along a substantially entire length L of the working channel tube 51. In other embodiments, the endoscope 1 can comprise two or more working channels and/or working channel tubes, in which case one or more of such further working channel tubes can be identical or different from any one of the embodiments as disclosed herein.

In the embodiments of FIGs 2a and 2b, respectively, the working channel 5 and working channel tube 51 each have a total or entire length L of about 1400 mm. In the embodiment of FIGs 3a and 3b, the working channel 5 and working channel tube 51 each have a total or entire length L of about 1700 mm. The working channel 5 and working channel tube 51 each extends from the entry port 44 of the working channel 5 to an exit port 58 in the tip part assembly 6. The working channel 5 and the working channel tube 51 each extends through an entire length of the insertion tube 3 and through the bending section.

The endoscope 1 as shown in FIG. 1a may be equipped with a light source and/or a vision receptor including a vision sensor of the camera assembly 61 positioned in the tip part assembly 6. Not shown electrical wiring for the camera and other electronics runs along an inside of the insertion tube 3 from the operating handle 4 to a PCB or FPC 65.

The working channel 5 can also be used as a suction channel, and the working channel tube 51 as a suction channel tube. In this case, the entry port 44 to the working channel 5 can be connected to a suction connector.

The tip part assembly 6 is connected to a distal end of the insertion tube 3. The insertion tube 3 connects the handle 4 to the bending section and to the tip part assembly 6. The working channel tube 51 extends from the handle 4, though the insertion tube 3, and to and through the tip part assembly 6 to the exit port 58 of the working channel 5.

The operating handle 4 can be gripped by an operator to operate the endoscope 1, potentially with one or two hands. The handle 4 comprises a handle housing 45 arranged at the proximal end of the insertion tube 3. The handle 4 is directly connected to the insertion tube 3.

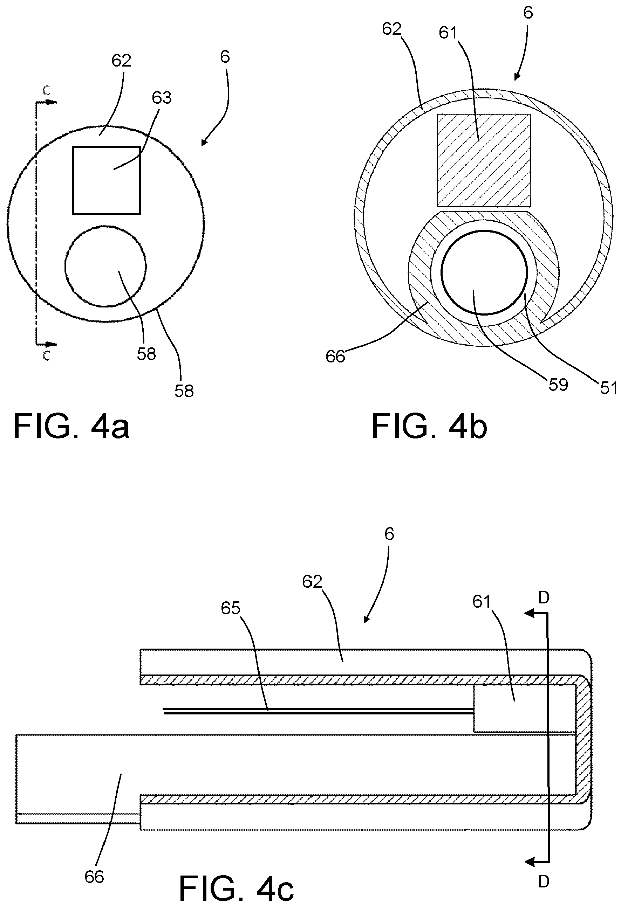

The tip part assembly 5 is provided at the distal end of the endoscope 1. At a proximal end (to the left in FIG. 4c), the tip part assembly 5 is connected to the not shown bending section and to a distal end of the insertion tube 3 via the bending section. A distal end (to the right in FIG. 4c) of the tip part assembly 8 forms a distal end of the endoscope 1.

Referring also to FIGS 4a to 5 and 1b, a camera assembly 6 is positioned in the tip part assembly 5 and is configured to capture images and transmit image signals via electrical wires to a not shown circuit board in the handle housing 45. A monitor cable 13, schematically shown with a dashed line in FIG. 1b, from a cable port 46 of the handle housing 45 to a cable port 21 of the monitor 2 connects the circuit board in the handle housing 45 with the monitor 2. FIG. 1b shows the monitor 2 having a display 22. The monitor 2 allows an operator of the endoscope 1 to view an image captured by the camera assembly 6 of the endoscope 1. The monitor 2 comprises the cable port 21 to which the monitor cable 13 can be connected to establish a signal communication between the camera assembly 6 of the endoscope 1 and the monitor 11. Thus, the endoscope 1, monitor 11, and cable 13 form parts of a system according to an embodiment of the systems of the present disclosure.

As schematically shown in FIGs 4b and 4c, the camera assembly 6 comprises the box-shaped camera housing 52 with a not shown camera module inside, the latter including an image sensor. The camera assembly 6 is at a proximal end thereof in a known manner connected to the schematically shown PCB/FPC 65. The camera assembly 6 is positioned above the working channel 5 in a top-down direction. The camera assembly 6 also includes a camera window 63. Provided in an end surface of the housing 62.

The housing 62 has a circumferentially extending outer surface for facing the environment. The outer surface encloses a volume and extends in a longitudinal direction between a proximal end and a distal end of the housing 62. Part of the working channel 5 is formed in one piece with the housing 62 and comprises the exit port or opening 58 in a distal surface of the housing 62. The camera assembly 61 is housed in the housing 62. The housing 62 is an outer or exterior housing for facing an exterior, flexible sleeve 7 and the environment. This sleeve 7 is shown in FIG. 1a and covers the bending section and extends to cover at least a part of the housing 62.

A proximal end of the housing 62 is in a not shown manner connected to a distal end segment of the bending section.

The working channel tube 5 is at the tip part assembly held in a circumferential and tubular working channel tube holder 66 for receiving and holding the working channel tube 51. This tube holder 66 has an internal diameter that corresponds to an outside diameter of the working channel tube 51, a distal end of the working channel tube 51 being positioned and held in the tube holder 66. The tube holder 66 is substantially rigid and is formed integral and in one piece with the housing 62. Both the housing 62 and the tube holder 66 include an open proximal end.

The endoscope 1 is of a type, which does not comprise an everting driving tube means and in which the working channel tube 51 does not contact any part of a patient during use of the endoscope 1. Rather, the insertion tube 3 shields or protects all parts of the working channel 5 and working channel tube 51 from the surroundings in any use position of the endoscope 1. The working channel tube 51 and the endoscope 1 are, thus, not of a type that everts during operation.

The working channel tube 51 and working channel spacing 59 extend from the handle 4 all the way to the distal end of the tip part assembly 6 and of the endoscope 1.

The working channel tube 51 of all three embodiments is a one-piece tube consisting of one single piece or length of tube that is not assembled from several pieces or lengths of tube, potentially from an endless tube.

The coil 56 of the embodiment of FIGs 3a and 3b can extend along an entire length of or only along a portion of the working channel tube 51, especially a portion at the distal end of the endoscope 1, such as a portion extending through the bending section and/or through the tip part assembly 6.

A method of manufacture of the endoscope 1 comprises coextruding the inner and outer layers 52, 53 of the working channel tube 51 to provide the working channel tube 51 and inserting the working channel tube 51 into the insertion tube 3 so that the working channel tube 51 establishes at least part of the working channel 5. The working channel tube 51 can potentially be cut from an endless coextruded tube having the structure of the working channel tube 51. At a proximal part of the working channel tube 51, an outer surface of the outer layer 53 can be glued or adhered to the operating handle 4. At a distal part of the working channel tube 51, an outer surface of the outer layer 53 can be glued or adhered to the housing 62 of the tip part assembly 6.

### List of references

The following is a list of reference numerals used throughout the present disclosure.
- 1: endoscope
- 12: cable socket
- 13: monitor cable
- 2: monitor
- 21: cable port
- 22: display
- 3: insertion tube
- 4: operating handle
- 41: control lever
- 42: control knob
- 43: control knob
- 44: entry port
- 45: housing
- 46: cable port
- 5: working channel
- 51: working channel tube
- 52: inner layer
- 52a: wall thickness of layer 52
- 53: outer layer
- 53a: wall thickness of layer 53
- 54: contact layer
- 54a: wall thickness of layer 54
- 55: total wall thickness of layers 52, 57
- 56: coil
- 57: tie layer
- 58: exit port
- 59: working channel spacing
- 6: tip part assembly
- 61: camera assembly
- 62: housing
- 63: camera window
- 65: PCB/FPC
- 66: tube holder
- 7: outer sleeve
- d: Inner diameter
- D: Outer diameter

## Claims

1. An endoscope comprising:
an elongate insertion tube; and
a working channel extending through the insertion tube to allow for insertion of a retractable instrument into a body and/or for suction of fluids from the body through the working channel;
wherein the working channel comprises an at least partly extruded or at least partly coextruded working channel tube, the working channel tube comprising an extruded inner layer of an inner layer material and an extruded outer layer of an outer layer material.

2. An endoscope according to claim 1, wherein the inner layer material is selected to provide a low surface friction of an inner surface of the inner layer towards the retractable instrument, and wherein the outer layer material is a support layer for prevention of kinking of the working channel tube.

3. An endoscope according to claim 1 or 2, wherein the outer layer material is a thermoplastic polyurethane.

4. An endoscope according to any one of the previous claims, wherein the outer layer material has a Shore A hardness according to ASTM D2240-15 within a range of 60 to 100.

5. An endoscope according to claim 4, wherein the outer layer material is a thermoplastic polyurethane having a Shore A hardness according to ASTM D2240-15 within a range of 85 to 100.

6. An endoscope according to any one of the previous claims, wherein the inner layer material is a high-density polyethylene.

7. An endoscope according to any one of the previous claims, wherein the outer layer has been extruded or extrusion coated on the inner layer or on a coextrusion comprising the inner layer so that the outer layer is attached to the inner layer or to the coextrusion comprising the inner layer.

8. An endoscope according to any one of claims 1 to 6, wherein the inner and outer layers have been coextruded with each other together with zero, one, or more intermediate layers, wherein one or more of the intermediate layers is optionally a coextrusion tie layer.

9. An endoscope according to any one of the previous claims, wherein a wall thickness of the inner layer is within a range of 0.01 to 0.1 mm, and a wall thickness of the outer layer is within a range of 0.1 to 0.5 mm.

10. An endoscope according to any one of the previous claims, wherein a wall thickness of the extruded and/or coextruded layers of the working channel tube is substantially the same along an entire length of the working channel tube.

11. An endoscope according to any one of the previous claims, wherein the working channel tube comprises a coil or spring positioned outside and/or inside and/or between extruded and/or coextruded layers of the working channel tube.

12. A method of manufacture of an endoscope according to any one of the previous claims, the method comprising
extruding or coextruding the inner and outer layers of the working channel tube to provide the working channel tube; and
inserting the working channel tube into the insertion tube so that the working channel tube establishes at least part of the working channel of the endoscope.

13. A method of manufacture of an endoscope,
the endoscope comprising a working channel extending through an elongate insertion tube to allow for insertion of a retractable instrument into a body and/or for suction of fluids from the body through the working channel,
wherein the method comprises:
extruding an inner layer material and an outer layer material to form a working channel tube of the endoscope, so that an inner layer of the working channel tube comprises the inner layer material and an outer layer of the working channel tube comprises the outer layer material,
inserting the working channel tube into the insertion tube so that the working channel tube establishes at least part of the working channel of the endoscope.

14. A method according to claim 12 or 13, wherein the manufactured endoscope is according to any one of claims 1 to 11.

15. A system for visually inspecting inaccessible places such as human body cavities, the system comprising an endoscope according to any one of claims 1 to 11 and a monitor or display.
